# EUROPEAN PATENT APPLICATION

(11) **EP 1 862 541 A1**
(43) Date of publication of application: **05.12.2007**
(21) Application number: 06290892.6
(22) Date of filing: 01.06.2006
(51) Int. Cl.: C12N 9/64, C12N 15/57, A61K 38/48

(54) **Polypeptides derived from the hemopexin-like domain of metalloproteinase MMP-2**

(71) Applicant: INSTITUT NATIONAL DE LA SANTE ET DE LA RECHERCHE MEDICALE (INSERM), 75013 Paris (FR)
(72) Inventor: Bikfalvi, Andreas, 33170 Gradignan (FR); Sala, Carlo, 20124 Milano (IT); Bello, Lorenzo, 10110 Torino (IT)
(74) Representative: Colombet, Alain André

(57) **Abstract**

The present invention relates to anti-angiogenic polypeptides derived from the non catalytic C-terminal hemopexin-like domain of the metalloproteinase MMP-2. The invention also provides pharmaceutical compositions comprising said anti-angiogenic polypeptides and methods for using said polypeptide for treating angiogenic diseases, and in particular for treating cancer.

SEQUENCE LISTING

<110>  INSERM

<120>  Polypeptides derived from the hemopexin-like domain of
       metalloproteinase MMP-2

<130>  BET06P0511

<160>  6

<170>  PatentIn version 3.3

<210>  1
<211>  210
<212>  PRT
<213>  Homo sapiens

<400>  1

Ile Asp Leu Gly Thr Gly Pro Thr Pro Thr Leu Gly Pro Val Thr Pro
1               5                   10                  15

Glu Ile Cys Lys Gln Asp Ile Val Phe Asp Gly Ile Ala Gln Ile Arg
            20                  25                  30

Gly Glu Ile Phe Phe Phe Lys Asp Arg Phe Ile Trp Arg Thr Val Thr
        35                  40                  45

Pro Arg Asp Lys Pro Met Gly Pro Leu Leu Val Ala Thr Phe Trp Pro
    50                  55                  60

Glu Leu Pro Glu Lys Ile Asp Ala Val Tyr Glu Ala Pro Gln Glu Glu
65                  70                  75                  80

Lys Ala Val Phe Phe Ala Gly Asn Glu Tyr Trp Ile Tyr Ser Ala Ser
                85                  90                  95

Thr Leu Glu Arg Gly Tyr Pro Lys Pro Leu Thr Ser Leu Gly Leu Pro
            100                 105                 110

Pro Asp Val Gln Arg Val Asp Ala Ala Phe Asn Trp Ser Lys Asn Lys
        115                 120                 125

Lys Thr Tyr Ile Phe Ala Gly Asp Lys Phe Trp Arg Tyr Asn Glu Val
    130                 135                 140

Lys Lys Lys Met Asp Pro Gly Phe Pro Lys Leu Ile Ala Asp Ala Trp
145                 150                 155                 160

Asn Ala Ile Pro Asp Asn Leu Asp Ala Val Val Asp Leu Gln Gly Gly

                165                 170                 175

Gly His Ser Tyr Phe Phe Lys Gly Ala Tyr Tyr Leu Lys Leu Glu Asn
            180                 185                 190

Gln Ser Leu Lys Ser Val Lys Phe Gly Ser Ile Lys Ser Asp Trp Leu
        195                 200                 205

Gly Cys
    210

<210> 2
<211> 237
<212> PRT
<213> Homo sapiens

<400> 2

Met Arg Gly Ser His His His His His His Gly Met Ala Ser Met Thr
1               5                   10                  15

Gly Gly Gln Gln Met Gly Arg Asp Leu Tyr Asp Asp Asp Asp Lys Asp
            20                  25                  30

Arg Trp Gly Ser Glu Leu Glu Pro Val Thr Pro Glu Ile Cys Lys Gln
        35                  40                  45

Asp Ile Val Phe Asp Gly Ile Ala Gln Ile Arg Gly Glu Ile Phe Phe
    50                  55                  60

Phe Lys Asp Arg Phe Ile Trp Arg Thr Val Thr Pro Arg Asp Lys Pro
65                  70                  75                  80

Met Gly Pro Leu Leu Val Ala Thr Phe Trp Pro Glu Leu Pro Glu Lys
                85                  90                  95

Ile Asp Ala Val Tyr Glu Ala Pro Gln Glu Glu Lys Ala Val Phe Phe
            100                 105                 110

Ala Gly Asn Glu Tyr Trp Ile Tyr Ser Ala Ser Thr Leu Glu Arg Gly
        115                 120                 125

Tyr Pro Lys Pro Leu Thr Ser Leu Gly Leu Pro Pro Asp Val Gln Arg
    130                 135                 140

Val Asp Ala Ala Phe Asn Trp Ser Lys Asn Lys Lys Thr Tyr Ile Phe
145                 150                 155                 160

Ala Gly Asp Lys Phe Trp Arg Tyr Asn Glu Val Lys Lys Lys Met Asp
                165                 170                 175

Pro Gly Phe Pro Lys Leu Ile Ala Asp Ala Trp Asn Ala Ile Pro Asp
            180                 185                 190

Asn Leu Asp Ala Val Val Asp Leu Gln Gly Gly Gly His Ser Tyr Phe
        195                 200                 205

Phe Lys Gly Ala Tyr Tyr Leu Lys Leu Glu Asn Gln Ser Leu Lys Ser
    210                 215                 220

Val Lys Phe Gly Ser Ile Lys Ser Asp Trp Leu Gly Cys
225                 230                 235

<210>  3
<211>  24
<212>  DNA
<213>  Artificial

<220>
<223>  primer

<400>  3
ccgctcgagc ctgtcactcc tgag                                              24

<210>  4
<211>  23
<212>  DNA
<213>  Artificial

<220>
<223>  primer

<400>  4
cggaatctca gcagcctagc cag                                               23

<210>  5
<211>  34
<212>  DNA
<213>  Artificial

<220>
<223>  primer

<400>  5
gagctcgagc ctgtcactcc tgagatctcc aaac                                   34

<210>  6
<211>  21
<212>  DNA
<213>  Artificial

<220>
<223>  primer

<400>  6
tcggaattct aaggagccta g                                                21

## Description

The present invention relates to anti-angiogenic polypeptides derived from the non catalytic C-terminal hemopexin-like domain of the metalloproteinase MMP-2. The invention also provides pharmaceutical compositions comprising said anti-angiogenic polypeptides and methods for using said polypeptide for treating angiogenic diseases, and in particular for treating cancer.

Angiogenesis is the fundamental process by which new blood vessels are formed and is essential to a variety of normal body activities, such as reproduction, development and wound repair in adult. The process is believed to proceed in any one of three ways: the vessels can sprout from pre-existing vessels, de-novo development of vessels can arise from precursor cells (vasculogenesis), or existing small vessels can enlarge in diameter. Angiogenesis requires the functional activity of a wide variety of molecules, including growth factors (VEGF, FGF), extracellular matrix proteins, adhesion receptors and proteolytic enzymes. During angiogenesis, the coordinated regulation of these proteins leads to endothelial cell proliferation, matrix remodeling, cellular migration/invasion, and eventually, differentiation. For instance, recent studies reported that angiogenesis depends on specific endothelial cell adhesive events mediated by integrin αvβ3 (Brooks, PC, et al., Science, 264: 569-571 (1994); Brooks, P.C, et al. Cell, 79: 1157-1164 (1994); Friedlander, M, et al., Science 270: 1500-1502 (1995)). Thus, the physiological control of angiogenesis is dependent on the balance of activators and inhibitors present within the vascular microenvironment.

Although angiogenesis is a highly regulated process under normal conditions, many diseases are driven by persistent unregulated angiogenesis. These clinical manifestations associated with angiogenesis are referred to as angiogenic diseases.

For instance, certain existing conditions such as arthritis, newly formed capillary blood vessels invade the joints and destroy cartilage. In diabetes, new capillaries formed in the retina invade the vitreous, bleed, and cause blindness.

Growth and metastasis of solid tumors are also angiogenic diseases (Folkman, J., Cancer Research, 46: 467-473 (1986)). It has been shown, for example, that tumors must obtain their own blood supply and do so by inducing the growth of new capillary blood vessels. Once these new blood vessels become embedded in the tumor, they provide a means for tumor cells to enter the circulation and metastasize to distant sites, such as liver, lung or bone (Weidner, N., et al., The New England Journal of Medicine, 324(1) : 1-8 (1991)).

Because angiogenic diseases impact a large number of people each year compositions and treatment methods for treating these diseases are highly desirable. From now on it is clear that blocking angiogenesis may be highly efficient treating angiogenic diseases. For example, there is great evidences supporting the contention that blocking tumor neovascularization can inhibit tumor growth in various animal models, and human clinical data is beginning to support this contention as well (Varner, J. A., Brooks, P. C., and Cheresh, D. A. (1995) Cell Adh. Commun. 3,367-374). Therefore several angiogenesis inhibitors are currently under development for use in treating angiogenic diseases.

A study provided evidence that the non catalytic C-terminal hemopexin like domain of metalloproteinase MMP-2, named PEX, can interact with integrin αvβ3 and thereby blocks MMP-2 binding to this integrin, resulting in a loss of cell-collagenolytic activity (Brooks PC, et al., Cell. Feb 6;92(3):391-400 (1998)). This study further showed that systemic administration of a recombinant form of PEX blocks angiogenesis and tumor growth in vivo. The capability of PEX for inhibiting angiogenesis has been confirmed in many studies (Pfeifer A, et al., Proc Natl Acad Sci U S A. 97(22):12227-32(2000); Bello L, et al., Cancer Res, 61(24):8730-6 (2001); Pluderi M, et al., J Neurosurg Sci., 47(2):69-78 (2003)).

Document WO 9745137 describes methods for inhibition of angiogenesis in tissues using integrin αvβ3 antagonists. In one embodiment described in this document, the antagonists are in the form of polypeptides, and in particular polypeptides derived from MMP-2 and in more particular relate to PEX polypeptide.

The present invention provides new mutants of the non catalytic C-terminal hemopexin-like domain of metalloproteinase MMP-2 (PEX) which have the same capability for inhibiting angiogenesis *in vitro* and *in vivo* as the wild type one, but can be more easily purified. Actually, the inventors have demonstrated that the disruption of the disulfide bond located between the cysteines in position 19 and 210 of PEX domain (when referred to sequence SEQ ID NO:1) has surprisingly no influence on the anti-angiogenic properties of PEX. Indeed, administration of said mutant to murine models of brain tumor leads to a significant extension of the lifespan. The inventors have also shown that said mutant is more soluble than the wild type polypeptide and therefore can be easily produced as a medicament intended for treating an angiogenic disease.

A first aspect of the invention thus relates to an anti-angiogenic polypeptide comprising the amino acid sequence ranging from positions 19 to 210 of SEQ ID NO:1, wherein at least one cysteine residue at position 19 or 210 of SEQ ID NO:1 is substituted or deleted, or a function-conservative variant thereof. In particular, said polypeptide may comprise the sequence ranging from positions 13 to 210 of SEQ ID NO:1, wherein at least one cysteine residue at position 19 or 210 of SEQ ID NO:1 is substituted or deleted, or a function-conservative variant thereof. Said polypeptide may also comprise the sequence SEQ ID NO: 1, wherein at least one cysteine residue at position 19 or 210 of SEQ ID NO:1 is substituted or deleted, or a function-conservative variant thereof. According to another embodiment, said polypeptide may comprise the amino acid sequence ranging from positions 35 to 237 of SEQ ID NO:2, wherein at least one cysteine residue at position 46 or 237 of SEQ ID NO:2 is substituted or deleted, or a function-conservative variant thereof. According to still another embodiment, said polypeptide may comprise the sequence SEQ ID NO:2, wherein at least one cysteine residue at position 46 or 237 of SEQ ID NO:2 is substituted or deleted, or a function-conservative variant thereof.

A second aspect of the invention relates to a nucleic acid encoding a polypeptide as above described.

A third aspect of the invention relates to a vector, which comprises a nucleic acid as above described.

A fourth aspect of the invention relates to a cell, which has been transfected, infected or transformed by a nucleic acid and/or a vector as above described.

A fifth aspect of the invention relates to a pharmaceutical composition, which is intended for inhibiting angiogenesis in an organism in need thereof, which comprises at least one polypeptide, one nucleic acid, one vector, or one cell as above described.

A sixth aspect of the invention relates to the use of a polypeptide as above described for the manufacture of a medicament intended for treating an angiogenic disease.

### Definitions

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

As used herein, the term "PEX" denotes the non catalytic C-terminal hemopexin like domain of metalloproteinase MMP-2. In the context of the invention PEX may originate from any mammalian species, including rodent, such as rat or mouse, feline, canine, primate, especially monkey or human. Preferably PEX is of human origin. The polypeptide sequence for PEX deposited in the database PDBSEQ under accession number 1 RTG is shown as SEQ ID NO:1.

The term "mPEX" or "MPEX" refers to a polypeptide comprising the sequence SEQ ID NO:1 wherein the two cysteine residues in positions 19 and 210 have been substituted by a serine residue.

An "anti-angiogenic polypeptide" denotes a polypeptide capable of inhibiting angiogenesis.

By "purified" and "isolated" it is meant, when referring to a polypeptide (i.e. the anti-angiogenic polypeptide of the invention) or a nucleotide sequence, that the indicated molecule is present in the substantial absence of other biological macromolecules of the same type. The term "purified" as used herein preferably means at least 75% by weight, more preferably at least 85% by weight, still preferably at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

"Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared.

Two amino acid sequences are "substantially homologous" or "substantially similar" when greater than 80 %, preferably greater than 85 %, preferably greater than 90 % of the amino acids are identical, or greater than about 90 %, preferably grater than 95 %, are similar (functionally identical). Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

The term "PEG" encompasses any polyethylene glycol molecule, without regard to size or to modification at an end of the PEG, and can be represented by the formula X-O(CH₂CH₂O)n-OH, where n is an integer between 20 and 2300, and X is H or a terminal modification, e.g., alkyl.

By "pegylation" is meant the process by which polyethylene glycol (PEG) chains are attached to polypeptides. The term "pegylated polypeptide" denotes a polypeptide that comprises at least one PEG group covalently conjugated to said polypeptide.

An "angiogenic disease" is a disease associated with unregulated angiogenesis.

As used herein, the term "subject" denotes a mammal, such as a rodent, a feline, a canine, and a primate. Preferably a subject according to the invention is a human.

### Anti-angiogenic polypeptides :

The invention thus provides an anti-angiogenic polypeptide comprising the amino acid sequence ranging from positions 19 to 210 of SEQ ID NO:1, wherein at least one cysteine residue at position 19 or 210 of SEQ ID NO:1 has been substituted or deleted, or a function-conservative variant thereof.

In particular, said polypeptide may comprise the amino acid sequence ranging from positions 13 to 210 of SEQ ID NO:1, wherein at least one cysteine residue at position 19 or 210 of SEQ ID NO:1 has been substituted or deleted, or a function-conservative variant thereof. Said polypeptide may also comprise the amino acid sequence SEQ ID NO:1, wherein at least one cysteine residue at position 19 or 210 of SEQ ID NO:1 has been substituted or deleted, or a function-conservative variant thereof.

Furthermore, the inventors have developed a modified PEX domain which sequence is shown as SEQ ID NO:2, in order to introduce a Histidine Tag, as well as *a Xhol* restriction site in the coding sequence. Accordingly, the polypeptide of the invention may comprise the amino acid sequence ranging from positions 35 to 237 of SEQ ID NO:2, wherein at least one cysteine residue at position 46 or 237 of SEQ ID NO:2 has been substituted or deleted, or a function-conservative variant thereof. The polypeptide may also comprise the amino acid sequence SEQ ID NO:2, wherein at least one cysteine residue at position 46 or 237 of SEQ ID NO:2 has been substituted or deleted, or a function-conservative variant thereof.

According to an embodiment, both cysteine residues are substituted or deleted altogether.

The cysteine residues may be substituted independently by a neutral amino acid selected from the group consisting of asparagine, glutamine, serine, threonine, tyrosine, glycine, alanine, valine, leucine, isoleucine, proline, phenylalanine, methionine and tryptophane. Preferably, the cysteine residues are independently substituted by an alanine or a serine residue. Still preferably, both cysteine residues are substituted by a serine residue.

According to an embodiment, the invention relates to the mPEX polypeptide itself, namely a polypeptide comprising the sequence SEQ ID N0:1 wherein the two cysteine residues at positions19 and 210 have been substituted by a serine residue.

The polypeptides of the invention may be produced by any technique known per se in the art, such as, without limitation, any chemical, biological, genetic or enzymatic technique, either alone or in combination.

Knowing the amino acid sequence of the desired sequence, one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Alternatively, the polypeptides of the invention can be synthesized by recombinant DNA techniques as is now well-known in the art. For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

Polypeptides of the invention can be use in an isolated (e.g., purified) form or contained in a vector, such as a membrane or lipid vesicle (e.g. a liposome).

### Nucleic acids, vectors and recombinant host cells

A further object of the invention relates to a nucleic acid comprising a sequence encoding a polypeptide of the invention.

Typically, said nucleic acid is a DNA or RNA molecule, which may be included in any suitable vector, such as a plasmid, cosmid, episome, artificial chromosome, phage or a viral vector. The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

So, a further object of the invention relates to a vector comprising a nucleic acid of the invention.

Such vectors may comprise regulatory elements, such as a promoter, enhancer, terminator and the like, to cause or direct expression of said polypeptide upon administration to a subject. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

Examples of plasmids include replicating plasmids comprising an origin of replication, or integrative plasmids, such as for instance pUC, pcDNA, pBR, and the like. Examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

A further object of the present invention relates to a cell which has been transfected, infected or transformed by a nucleic acid and/or a vector according to the invention. The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids of the invention may be used to produce a recombinant polypeptide of the invention in a suitable expression system. The term "expression system" means a host cell and compatible vector under suitable conditions, e.g. for the expression of a protein coded for by foreign DNA carried by the vector and introduced to the host cell.

Common expression systems include *E. coli* host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include *E.coli, Kluyveromyces* or *Saccharomyces* yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). More particularly, the invention contemplates vascular or endothelial cells thereof or derived thereof, such as human umbilical vein endothelial (HUVEC) or progenitor endothelial cells (PEC).

The present invention also relates to a method for producing a recombinant host cell expressing an anti-angiogenic polypeptide according to the invention, said method comprising the steps consisting of : (i) introducing *in vitro* or *ex vivo* a recombinant nucleic acid or a vector as described above into a competent host cell, (ii) culturing *in vitro* or *ex vivo* the recombinant host cell obtained and (iii), optionally, selecting the cells which express and/or secrete said anti-angiogenic polypeptide. Such recombinant host cells can be used for the production of anti-angiogenic polypeptides according to the present invention, as previously described.

The invention further relates to a method of producing an anti-angiogenic polypeptide according to the invention, which method comprises the steps consisting of: (i) culturing a transformed host cell according to the invention under conditions suitable to allow expression of said anti-angiogenic polypeptide; and (ii) recovering the expressed polypeptide.

### Function-conservative variants

A further object of the present invention encompasses function-conservative variants of the anti-angiogenic polypeptides of the present invention.

Modifications and changes may be made in the structure of the polypeptides of the present invention, and in the DNA sequences encoding them, and still obtain a functional molecule that encodes a polypeptide with desirable characteristics. Nevertheless, said function-conservative variants shall not contain any disulfide bridge and therefore comprise a substitution or a deletion of at least one of the cysteine residues, as compared with the sequence of PEX domain as shown in SEQ ID NO:1.

The amino acid changes may be achieved by changing codons in the DNA sequence, according to Table 1.

**Table 1**

| **Amino acids** | | | **Codons** |
|---|---|---|---|
| Alanine | Ala | A | GCA, GCC, GCG, GCU |
| Cysteine | Cys | C | UGC, UGU |
| Aspartic Acid | Asp | D | GAC, GAU |
| Glutamic acid | Glu | E | GAA, GAG |
| Phenylalanine | Phe | F | UUC, UUU |
| Glycine | Gly | G | GGA, GGC, GGG, GGU |
| Histidine | His | H | CAC, CAU |
| Isoleucine | Ile | I | AUA, AUC, AUU |
| Lysine | Lys | K | AAA, AAG |
| Leucine | Leu | L | UUA, UUG, CUA, CUC, CUG, CUU |
| Methionine | Met | M | AUG |
| Asparagine | Asn | N | AAC, AAU |
| Proline | Pro | P | CCA, CCC, CCG, CCU |
| Glutamine | Gln | Q | CAA, CAG |
| Arginine | Arg | R | AGA, AGG, CGA, CGC, CGG, CGU |
| Serine | Ser | S | AGC, AGU, UCA, UCC, UCG, UCU |
| Threonine | Thr | T | ACA, ACC, ACG, ACU |
| Valine | Val | V | GUA, GUC, GUG, GUU |
| Tryptophan | Trp | W | UGG |
| Tyrosine | Tyr | Y | UAU |

For example, certain amino acids may be substituted for other amino acids in a protein structure without appreciable loss of anti-angiogenic capability. Since it is the interactive capacity and nature of a protein that defines that protein's biological functional activity, certain amino acid substitutions can be made in a protein sequence, and, of course, in its DNA encoding sequence, and nevertheless obtain a protein with like properties. It is thus contemplated that various changes may be made in the polypeptide sequences of the invention, or corresponding DNA sequences which encode said polypeptides, without appreciable loss of their biological activity.

Said anti-angiogenic activity can be assessed by different techniques well-known in the art as described hereinafter.

In making the changes in the amino sequences of polypeptide, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3. 8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (<RTI3.5) ; asparagine (-3.5); lysine (-3.9); and arginine (-4.5).

It is known in the art that certain amino acids may be substituted by other amino acids having a similar hydropathic index or score and still result in a protein with similar biological activity, i.e. still obtain a biological functionally equivalent protein.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

The anti-angiogenic activity of the function-conservative variants may be assessed according any method of assaying anti-angiogenic activity known in the art, such as for instance the assays referred in the instant application.

### Pegylation

Pegylation is a well established and validated approach for the modification of a range of polypeptides (Chapman, A., Adv. Drug Deliv. Rev. 54:531-545, 2002). The benefits include among others : (a) markedly improved circulating half-lives *in vivo* due to either evasion of renal clearance as a result of the polymer increasing the apparent size of the molecule to above the glomerular filtration limit, and/or through evasion of cellular clearance mechanisms; (b) reduced antigenicity and immunogenicity of the molecule to which PEG is attached; (c) improved pharmacokinetics; (d) enhanced proteolytic resistance of the conjugated protein (Cunningham-Rundles et.al., J. Immunol. Meth. 152:177-190, 1992); and (e) improved thermal and mechanical stability of the PEGylated polypeptide.

Therefore, advantageously, the polypeptides of the invention may be covalently linked with one or more polyethylene glycol (PEG) group(s).

Accordingly, one aspect of the invention provides modified polypeptides, wherein the modification comprises a single polyethylene glycol group covalently conjugated to the polypeptide. Other aspects provide modified polypeptides covalently conjugated to one, two, three, or more polyethylene glycol groups. The one or more PEG may have a molecular weight ranging from about 1 kDa to about 100 kDa, and will preferably have a molecular weight ranging from about 10 to about 60 kDa or about 10 to about 40 kDa. One skilled in the art can select a suitable molecular mass for PEG, based on how the pegylated polypeptide will be used therapeutically by considering different factors including desired dosage, circulation time, resistance to proteolysis, immunogenicity, etc.

In one embodiment, the PEG of the invention terminates on one end with hydroxy or methoxy, i.e., X is H or CH₃ ("methoxy PEG"). In addition, such a PEG can consist of one or more PEG side-chains which are linked together. PEGs with more than one PEG chain are called branched PEGs. Branched PEGs can be prepared, for example, by the addition of polyethylene oxide to various polyols, including glycerol, pentaerythriol, and sorbitol. For example, a four-armed branched PEG can be prepared from pentaerythriol and ethylene oxide. One form of PEGs includes two PEG side-chains (PEG2) linked via the primary amino groups of a lysine (Monfardini, C, et al., Bioconjugate Chem. 6 (1995) 62-69).

To effect covalent attachment of PEG groups to the polypeptide, the hydroxyl end groups of the polymer molecule must be provided in activated form, i. e. with reactive functional groups (examples of which include primary amino groups, hydrazide (HZ), thiol, succinate (SUC), succinimidyl succinate (SS), succinimidyl succinamide (SSA), succinimidyl proprionate (SPA), succinimidy carboxymethylate (SCM),benzotriazole carbonate (BTC), N-hydroxysuccinimide (NHS), aldehyde, nitrophenylcarbonate (NPC), andtresylate (TRES)). Suitable activated polymer molecules are commercially available, e. g. from Shearwater Polymers, Inc., Huntsville, AL, USA, or from PolyMASC Pharmaceuticals plc, UK. Alternatively, the polymer molecules can be activated by conventional methods known in the art, e. g. as disclosed in WO 90/13540. Specific examples of activated linear or branched polymer molecules for use in the present invention are described in the Shearwater Polymers, Inc. 1997 and 2000 Catalogs (Functionalized Biocompatible Polymers for Research and pharmaceuticals, Polyethylene Glycol and Derivatives, incorporated herein by reference). Specific examples of activated PEG polymers include the following linear PEGs : NHS-PEG (e.g. SPA-PEG, SSPA-PEG, SBA-PEG, SS-PEG, SSA-PEG, SC-PEG, SG-PEG, and SCM-PEG), and NOR-PEG, BTC-PEG, EPOX-PEG, NCO-PEG, NPC-PEG,CDI-PEG, ALD-PEG, TRES-PEG, VS-PEG,IODO-PEG, and MAL-PEG, and branched PEGs such as PEG2-NHS.

The conjugation of the polypeptide and the activated polymer molecules is conducted by use of any conventional method. Conventional methods are known to the skilled artisan. The skilled person will be aware that the activation method and/or conjugation chemistry to be used depends on the attachment group(s) of the polypeptides as well as the functional groups of the PEG molecule (e.g., being amine, hydroxyl, carboxyl, aldehyde, ketone, sulfhydryl, succinimidyl, maleimide, vinylsulfone or haloacetate).

In one embodiment, polypeptides are conjugated with PEGs at amino acid D and E (for COOH), T, Y and S (for OH), K (for NH₂), C (for SH if at least one cysteine is conserved) or/and Q et N (for the amide function).

In one embodiment, additional sites for PEGylation can be introduced by site-directed mutagenesis by introducing one or more lysine residues. For instance, one or more arginine residues may be mutated to a lysine residue. In another embodiment, additional PEGylation sites are chemically introduced by modifying amino acids on polypeptides of the invention.

In one embodiment, PEGs are conjugated to the polypeptide through a linker. Suitable linkers are well known to the skilled person. A preferred example is cyanuric chloride (Abuchowski et al., (1977), J. Biol. Chem., 252,3578-3581 ; US 4,179, 337).

Conventional separation and purification techniques known in the art can be used to purify pegylated polypeptides of the invention, such as size exclusion (e.g. gel filtration) and ion exchange chromatography. Products may also be separated using SDS-PAGE.

In one embodiment, the pegylated polypeptides provided by the invention have a serum half-life in vivo at least 50%, 75%, 100%, 150% or 200% greater than that of an unmodified polypeptide.

### Vascular or tumor targeting agent:

In another embodiment the polypeptides of the invention are conjugated to a vascular or tumor targeting agent.

Said vascular and/or tumor targeting agents include but are not limited to antibodies directed against the EDB domain of fibronectin, antibodies or agents binding Vascular endothelial growth factor receptor 2, antibodies or molecules binding fibroblast growth factor receptor-1, antibodies or agents that interact with CD31, antibodies or agents interacting with tumor lymphatic endothelium (Podoplanin, Lyve-1), or antibodies or agents binding to αVβ3 integrin such as RGD peptides. Strategies for vascular targeting in tumors have been reviewed for instance by Brekken et al. (int. J. Cancer. 2002;100 (2): 123-130).

### Angiogenic diseases

Angiogenic diseases include but are not limited to primary and metastatic solid tumors, including carcinomas of breast, colon, rectum, lung, oropharynx, hypopharynx, esophagus, stomach, pancreas, liver, gallbladder and bile ducts, small intestine, kidney, bladder, urothelium, female genital tract, (including cervix, uterus, and ovaries as well as choriocarcinoma and gestational trophoblastic disease), male genital tract (including prostate, seminal vesicles, testes and germ cell tumors), endocrine glands (including the thyroid, adrenal, and pituitary glands), and skin, as well as hemangiomas, melanomas, sarcomas (including those arising from bone and soft tissues as well as Kaposi's sarcoma) and tumors of the brain, nerves, eyes, such as astrocytomas, gliomas, glioblastomas, retinoblastomas, neuromas, neuroblastomas, Schwannomas, and meningiomas.

Angiogenic diseases also relate to tumors arising from hematopoietic malignancies such as leukemias as well both Hodgkin's and non-Hodgkin's lymphomas.

Angiogenic diseases also pertain to rheumatoid, immune and degenerative arthritis; various ocular diseases such as diabetic retinopathy, retinopathy of prematurity, corneal graft rejection, retrolental fibroplasia, neovascular glaucoma, rubeosis, retinal neovascularization due to macular degeneration, hypoxia, angiogenesis in the eye associated with infection or surgical intervention, and other abnormal neovascularization conditions of the eye.

Angiogenic diseases further include skin diseases such as psoriasis; blood vessel diseases such as hemagiomas, and capillary proliferation within atherosclerotic plaques;Osler-Webber Syndrome; myocardial angiogenesis; plaque neovascularization; telangiectasia; hemophiliacjoints' ; angiofibroma; and wound granulation.

Other angiogenic diseases include diseases characterized by excessive or abnormal stimulation of endothelial cells, including but not limited to intestinal adhesions, Crohn's disease, atherosclerosis, scleroderma, and hypertrophic scars, i.e. keloids., diseases that have angiogenesis as a pathologic consequence such as cat scratch disease (Rochele ninalia quintosa) and ulcers (Helicobacter pylori).

### Therapeutic methods and uses

In one embodiment, the invention provides a method for treating an angiogenic disease comprising administering a subject in need thereof with a therapeutically effective amount of a polypeptide or nucleic acid of the invention.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the term "patient" or "patient in need thereof', is intended for a human or non-human mammal affected or likely to be affected with an angiogenic disease.

By a "therapeutically effective amount" of the polypeptide of the invention is meant a sufficient amount of the polypeptide to treat an angiogenic disease, (for example, to limit tumor growth or to slow or block tumor metastasis) at a reasonable benefit/risk ratio applicable to any medical treatment. It will be understood, however, that the total daily usage of the polypeptides and compositions of the present invention will be decided by the attending physician within the scope of sound medical judgment. The specific therapeutically effective dose level for any particular patient will depend upon a variety of factors including the disorder being treated and the severity of the disorder; activity of the specific polypeptide employed; the specific composition employed, the age, body weight, general health, sex and diet of the patient; the time of administration, route of administration, and rate of excretion of the specific polypeptide employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved.

In another one embodiment the invention relates to the use of at least one polypeptide or nucleic acid of the invention for the manufacture of a medicament intended for treating an angiogenic disease.

### Pharmaceutical compositions

The polypeptides or nucleic acid of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular. entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the anti-angiogenic polypeptide of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An anti-angiogenic polypeptide of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The anti-angiogenic polypeptide may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the polypeptides formulated for parenteral administration, such as intravenous or intramuscular injection, other pharmaceutically acceptable forms include, e.g. tablets or other solids for oral administration ; liposomal formulations ; time release capsules ; and any other form currently used.

A polypeptide of the invention can be delivered in a pharmaceutically acceptable ophthalmic vehicle, such that the polypeptide can penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically-acceptable ophthalmic vehicle may, for example, be an ointment, vegetable oil or an encapsulating material. Alternatively, the polypeptides of the invention may be injected directly into the vitreous, aqueous humour, ciliary body tissue(s) or cells and/or extra-ocular muscles by electroporation means.

The polypeptides of the invention may also be combined with other anti-angiogenic agents to enhance their effectiveness, or combined with other anti-angiogenic agents and administered together with other cytotoxic agents. In particular, when used in the treatment of solid tumors, polypeptides of the invention may be administered with IL-12, retinoids, interferons, angiostatin, endostatin, thalidomide,thrombospondin-1, thrombospondin-2, captopryl, antineoplastic agents such as alpha inteferon, COMP (cyclophosphamide, vincristine, methotrexate and prednisone), etoposide, mBACOD (methortrexate, bleomycin, doxorubicin, cyclophosphamide, vincristine and dexamethasone), PRO-MACE/MOPP (prednisone, methotrexate (w/leucovin rescue), doxorubicin, cyclophosphamide,taxol, etoposide/mechlorethamine, vincristine, prednisone and procarbazine), vincristine, vinblastine, angioinhibins, TNP-470, pentosan polysulfate, platelet factor 4, angiostatin, LM609, SU-101, CM-101,Techgalan, thalidomide, SP-PG and the like as well as with rediation.

In an particular embodiment, the invention encompasses composition comprising a polypeptide of the invention in combination with an other anti-angiogenic polypeptide chosen from the group comprising PF-4, fragments and fusion peptides derived from PF-4, as those described in W00206300.

### Anti-angiogenic assays

The polypeptides of the invention, and especially function-conservative variants of the invention, can be assayed for their ability to inhibit angiogenesis. Any suitable assay known to one of skill in the art can be used to monitor such effects. Several such techniques are described herein.

For example, one assay measures angiogenesis in the chick chorioallantoic membrane (CAM) and is referred to as the CAM assay. The CAM assay has been described in detail by others, and further has been used to measure both angiogenesis and neovascularization of tumor tissues (Ausprunk et al., Am. J. Pathol, 79:597-618 (1975) and Ossowski et al., Cancer Res., 40:2300-2309 (1980)). The CAM assay is a well recognized assay model for in vivo angiogenesis because neovascularization of whole tissue is occurring, and actual chick embryo blood vessels are growing into the CAM or into the tissue grown on the CAM. Furthermore, it is easy to monitor the growth of any tissue transplanted upon the CAM, such as a tumor tissue. Finally, the assay is particularly useful because there is an internal control for toxicity in the assay system. The chick embryo is exposed to any test reagent, and therefore the health of the embryo is an indication of toxicity.

A second assay measuring angiogenesis is the *in vivo* rabbit eye model and is referred to as the "rabbit eye assay". The rabbit eye assay has been described in details by others, and further has been used to measure both angiogenesis and neovascularization in the presence of angiogenic inhibitors such as thalidomide (D'Amato et al. (1994) Proc. Natl. Acad. Sci. 91:4082-4085). The rabbit eye assay is a well recognized assay model for *in vivo* angiogenesis because the neovascularization process, exemplified by rabbit blood vessels growing from the rim of the cornea into the cornea, is easily visualized through the naturally transparent cornea of the eye. Additionally, both the extent and the amount of stimulation or inhibition of neovascularization or regression of neovascularization can easily be monitored over time. Finally, the rabbit is exposed to any test reagent, and therefore the health of the rabbit is an indication of toxicity of the test reagent.

A third assay measures angiogenesis in the chimeric mouse:human mouse model and is referred to as the "chimeric mouse assay". The assay has been described in details by others, and further has been described herein to measure angiogenesis, neovascularization, and regression of tumor tissues (Yan, et al. (1993) J. Clin. Invest. 91:986-996). The chimeric mouse assay is a useful assay model for *in* vivo angiogenesis because the transplanted skin grafts closely resemble normal human skin histologically and neovascularization of whole tissue is occurring wherein actual human blood vessels are growing from the grafted human skin into the human tumor tissue on the surface of the grafted human skin. The origin of the neovascularization into the human graft can be demonstrated by immunohistochemical staining of the neovasculature with human-specific endothelial cell markers. The chimeric mouse assay demonstrates regression of neovascularization based on both the amount and extent of regression of new vessel growth. Furthermore, it is easy to monitor effects on the growth of any tissue transplanted upon the grafted skin, such as a tumor tissue. Finally, the assay is useful because there is an internal control for toxicity in the assay system. The chimeric mouse is exposed to any test reagent, and therefore the health of the mouse is an indication of toxicity.

A fourth assay measures angiogenesis in an orthotopic tumor model (Bello et al. Cancer Treat Res. 2004;117:263-84). For example, for brain malignancies, brain tumor cells are implanted into the mouse brain and growth and angiogenesis is monitored in short term or long term experiments. After a specified time point, brains are analysed for vessel density, the presence of vessel associated pericytes, tumor cell proliferation and apoptosis.

In a fifth assay, tumor development and angiogenesis are measured in transgenic mouse tumor models such as the Rip Tag or Tyrp-Tag transgenic mice (Rousseau et al Cancer Res. 2004 Apr 1;64(7):2490-5).

The invention will further be illustrated in view of the following figures and examples.

### FIGURES

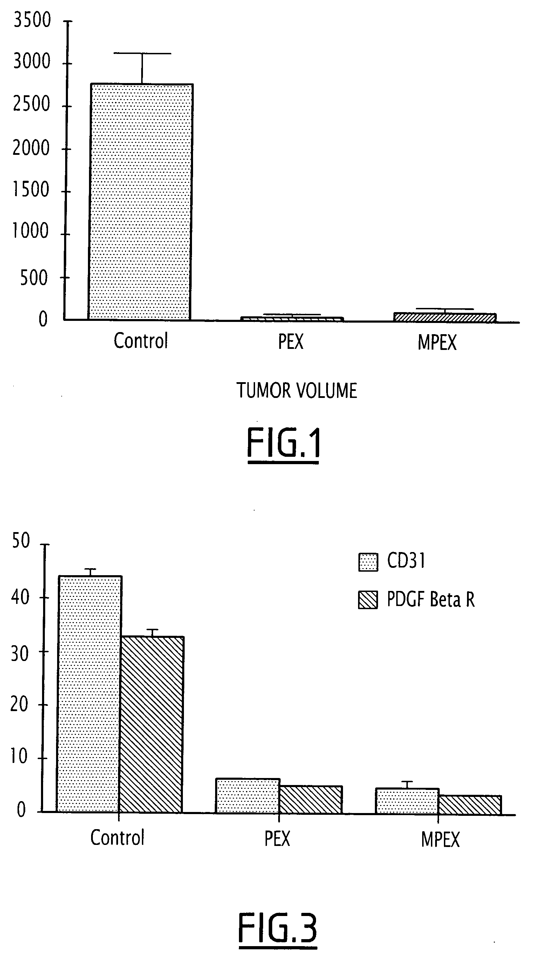

Figure 1 : Short-term experiment on intracranial U87 growth in the presence of mPEX or PEX
Figure 2: Immunohistology of short-term experiments of intracranial U87 growth (CD31 Staining). Measurement of vessel count, vessel density and intercapillary distance. PEX and mPEX decrease both the vessel count and vessel diameter but increase the intercapilarry distance.
Figure 3: Factor VIII and PDGF-beta receptor expression in histological section of U87 tumors in mice treated with PEX or mPEX. Both molecules decreases similarly double positive PDGF-beta receptor /factor VIII vessels.
Figure 4: Desmin and NG2 expression in histological section of U87 tumors in mice treated with PEX or mPEX. Both molecules decrease similarily desmin and NG2 expression. This indicates that mPEX and PEX are inhibiting pericyte recruitment similarily.
Figure 5: Ki67 expression and TUNEL in histological section of U87 tumors in mice treated with PEX or mPEX. Both molecules decrease Ki67 (proliferation) and increase the apoptotic indexes (TUNEL).
Figure 6: Collagen IV expression in histological section of U87 tumors in mice treated with PEX or mPEX. Both molecules decrease collagen IV expression.
Figure 7: Effect of mPEX and PEX in the Tyrp Tag transgenic mouse model. Both molecules inhibit the growth and invasion of tumors derived from the retinal pigmented epithelium of the eye, into the brain.
Figure 8: Long-term survival experiments in mice implanted intracranially with U87 glioma (Kaplan-Meyer analysis). Both molecules increase survival similarly.

### EXAMPLES :

### Example 1: Experimental procedures

**Production of recombinant PEX and MPEX** : PEX RNA was amplified from U87 glioblastoma cells by the Polymerase Chain Reaction using the following primers: 5' CCGCTCGAGCCTGTCACTCCTGAG 3' (sense, SEQ ID NO:3) and 5' CGGAATCTCAGCAGCCTAGCCAG 3' (anti sense, SEQ ID NO:4). The fragment was cloned into the pRSET vector (Invitrogen, Carlsbad, CA) and transformed in BL21 bacteria. Transformed BL21 bacteria were grown in LB media followed by induction with 1 mM isopropylthiolgalactoside (IPTG). PEX was purified under denaturing conditions since the majority of the protein was found in inclusion bodies. PEX was then purified by Ni-charged chelating agarose (Ni-NTA agarose). Recombinant protein was refolded and dialyzed against water and the protein concentration was determined. Purity of the preparation was confirmed by running the purified protein on a 12% SDS PAGE gel.

Cys19Ser and Cys210Ser (when referred to sequence SEQ ID NO:1) or Cys46 and Cys237 (when referred to sequence SEQ ID NO:2) mutated PEX was generated using wtPEX as a template and the following primers :
Primer 1 : 5' GAGCTCGAGCCTGTCACTCCTGAGATCTCCAAAC 3' (SEQ ID NO:5);
Primer 2 : 5' TCGGAATTCTAAGGAGCCTAG 3' (SEQ ID NO:6).

The PCR product was subcloned into a PGEM-T vector and the mutated PEX (mPEX) cDNA obtained after cutting with Xhol and EcoRl and purification. The mPEX cDNA was then subloned into the pRSET vector at Xhol and EcoRl sites. Transformed bacteria were then induced with IPTG and mPEX purified as previously described.The biological activity of PEX and MPEX were tested in vitro using angiogenic and proliferation assays.

**Cell cultures** : U87-MG cells (ATCC, MD) were used in the animal experiments. U87-MG cells were cultured in MEM alpha supplemented with 2 mM L-glutamine and 10% FBS. Media were supplemented with 1,000 U/ml penicillin/streptomycin solution, and the cells cultured in a 5% C02 incubator at 37°C.

**Western Blotting :** For Western Blot analysis, samples were boiled and analysed by SDS-PAGE. Protein samples were electrophoretically blotted onto Immobilon-P membranes (Millipore, Bedford, MA) and blocked with 5% non-fat milk in Tris buffered saline (TBS, 10 mM Tris, pH 8.00, 0.9% NaCl) containing 0.1% Tween-20 (TBS-T) for 1 hr. Blots were incubated for 1 hr at RT with the primary antibody (human PEX: Monoclonal Antibody IM3LL anti-MMP-2, 1:100 dilution in PBS; Calbiochem, La Jolla, CA). The blots were then incubated with secondary antimouse or anti-rabbit horseradish peroxidase conjugate antibody (1:1500 in PBS) for 1 hour at RT. Detection of antibodies was performed using the ECL-Plus system (Amersham Life Science).

### Animal studies

### A) Short-term combinations experiments

In this set of experiments, groups of 10 six-week old male nude mice were implanted with 50,000 U87 or GL261 glioblastoma cells. Twelve days after tumor cell injection, animals were implanted with subcutaneous Alzet 2004 osmotic minipumps which reservoir was filled with human PEX, or MPEX at the total concentration of 0.5 mg which corresponds to 0.5 mg/Kg/day, or PBS. The last group of mice was used as control. In a second set of experiments, 4 week-old Tyrp1-TagSV40T mice were implanted with subcutaneous Alzet 2004 osmotic minipumps filled with similar concentration of human PEX, MPEX, or PBS. In both experimental conditions, treatment was continued for 28 days, corresponding to the working period of the pumps. Animals were sacrificed at the occurrence of any side effect or neurological distress, and in any case 29 days after pump implantation. At sacrifice, brains were removed, fixed in 5% paraformaldeyde in PBS for 24 hrs at 4°C, dehydrated in 30% sucrose in PBS for 24 hrs at 4 °C, embedded in OCT (Tissue-Tek; Miles, Elkhart, IN) and stored at -70°C. The brains were then sectioned, and a portion of them submitted to routine histological examination with H&E (Hematoxylin/Eosine) staining. Tumor volume was calculated, and expressed as a mean ± S.E. Tumor volume was estimated using the formula for ellipsoid (width 2 x length)/2. The remaining slides were used for the immunohistochemistry analysis as described below. Statistical analysis of tumor volumes was performed with a two-way ANOVA. Pairwise comparison between treatment groups at each dose was performed by the Newman-Keuls post-test.

### B) Long-term experiments

In this set of experiments, groups of 10 six-week old male nude mice were implanted with 50,000 U87 cells. Twelve days after tumor cell injection, animals were implanted with Alzet 2004 osmotic minipumps which reservoir was filled with human PEX, or MPEX at the concentration of 0.5 mg/Kg/day, or PBS. The pump reservoirs were changed three times in order to afford a treatment period of 110 days. Animals were sacrificed at the occurrence of any side effects or neurological distress, and in any case 110 days after tumor cell implantation. At sacrifice, the brains were removed and processed as previously described. Kaplan-Meyer survival curves were designed, and survival for treated and untreated mice compared with log-rank test. All animal experiments were repeated at least two times.

**Immunohistochemical and immunofluorescence analysis** : For immunohistochemical studies, specimens were embedded in OCT (Tissue-Tek; Miles, Elkhart, IN), frozen on dry ice/butane and stored at -80°C. Frozen sections (6 µm) were cut using a cryostat (ICE Microtome). Sections of each specimen were stained using Hematoxylin and Eosin. Tumor volumes were calculated using the ellipsoid model. Primary antibodies included CD31 (B&D, Pharmigen, San Jose, CA), CD34 (MCA1825, Serotec, England), desmin (Dako, Carpinteria, CA), S smooth muscle AC (Sigma), PDGFBeta receptor (CD140b, Bioscience), NG2 (Chemicon) and Ki-67 nuclear antigen (Dako, Carpinteria, CA), collagen IV (Chemicon), HIF-1 alpha (Chemicon). L19 for the detection of the EDB fibronectin domain was provided by Luciano Zardi (Istituto Ricerca sul Cancro, Genova) and Dario Neri (Swiss Federal Institute of Technology (Kaspar M, Zardi L, Neri D. Fibronectin as target for tumor therapy. Int J Cancer. 2006 Mar 15;118(6):1331-9.). Immunohistochemistry was carried out using the Vectastain Elite ABC kit (Vector Laboratories, Burlingame, CA). Detection was carried out using a DAB chromogen, which resulted in a positive brown staining. For immunofluorescence studies, 10 µm sections was fixed in 5%PAF in PBS, ethanol, or cold acetone. Secondary antibodies include anti rabbit and anti rat Texas red and Fluorescein conjugated (Vector Laboratories), or anti rabbit Cyan 5 (Bioscience). Sections were counterstained with DAPI (6-12). To detect tumor hypoxia, 60 mg/Kg pimonidazole was injected iv 1 hr before brains was rapidly frozen. The hypoxyprobe-1 kit plus (Chemicon) was used to detect pimonidazole-protein adducts in tumor sections (21). Sections were counterstained with hematoxylin. Ki-67 staining was quantified by counting the number of positively stained cells of all nuclei in 20 randomly chosen fields. Quantification of vessel density and count were performed on different regions of the tumors (tumor periphery, tumor center) in at least 5 different slides for each region, and on at least 15 fields for each specimen, on CD31 stained sections. Measurement of vessel diameters were performed on the same sections and fields with the aid of a computer Software (NIH Image 1.63) on a Power Book G4 Macintosh computer. Measurements of basal membrane thickness and EDB staining was performed on immunofluorescence sections in different regions of the tumors, in at least 5 different slides for each region, and on at least 15 fields for each specimen, double stained for CD31 and collagen IV, or EDB fibronectin domain and the aid of NIH 1.63 software and Photoshop software. Quantification of alpha SMA, desmin, PDGFBetaReceptor, or NG2 positive cells, was performed on immunofluorescence sections in different regions of the tumors, in at least 5 different slides for each region, and on at least 15 fields for each specimen, double stained for CD31 and alpha SMA, desmin, PFGFBeta Receptor, or NG2, and the aid of NIH 1.63 software and Photoshop software. Data will be expressed as mean +/- SD.

**Apoptosis assay :** In situ detection of apoptosis was also measured by the TUNEL-method (terminal deoxinucleotidyltransferase-mediated d-UTP-biotin nick end-labeling) using the ApopTag Plus Kit (Oncor, Gaithersburg) followed by counterstaining with 1 % methyl green. Apoptosis was quantified by determining the percentage of positively stained cells for all nuclei in 20 randomly chosen fields per section at 200x magnification.

### Example 2: Results

### Production of human PEX or MPEX :

Two mgs of human PEX were extracted from 10 L of bacteria media. Under similar conditions 20 mgs of MPEX were extracted from the same volume of media. Thus MPEX production is characterized by a ten-fold increase in yield following protein extraction. Both molecules appeared as a single band at 28 kDa in the Western Blot analysis.

### Short and long term experiments

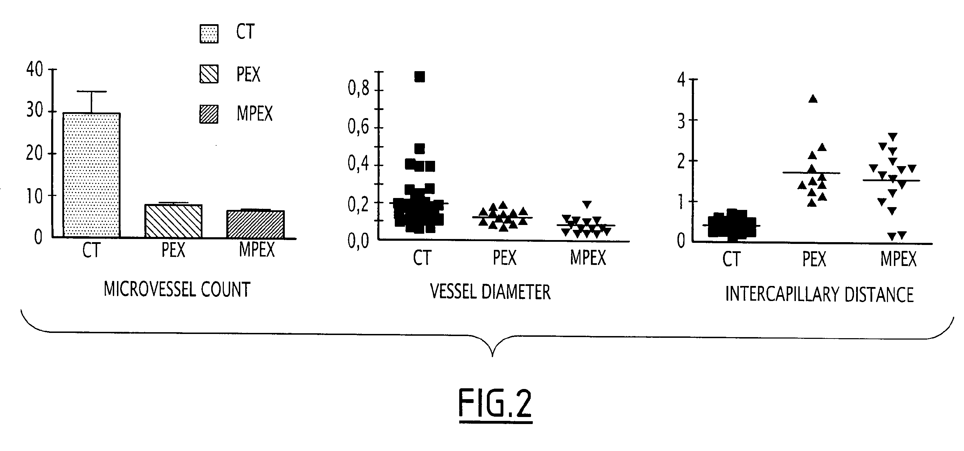

The effect of MPEX on glioma growth in vivo was initially investigated by performing short-term studies in U87 glioma cell model in nude mice and compared to those induced by human PEX. In these studies, the inhibitors were administered continuously and systemically, by the use of osmotic minipumps implanted subcutaneously in the right flank of the animals. The pumps were implanted 12 days after tumor cell injection. Animals were sacrificed after 29 days from the implantation of the pumps, at the end of their working period. After the sacrifice, the brains were removed, fixed, sectioned and the tumor volumes were calculated. Treatment with PEX and MPEX both resulted in a potent inhibition of U87 glioma growth (98% and 97%) tumor volume inhibition respectively (Figure 2).

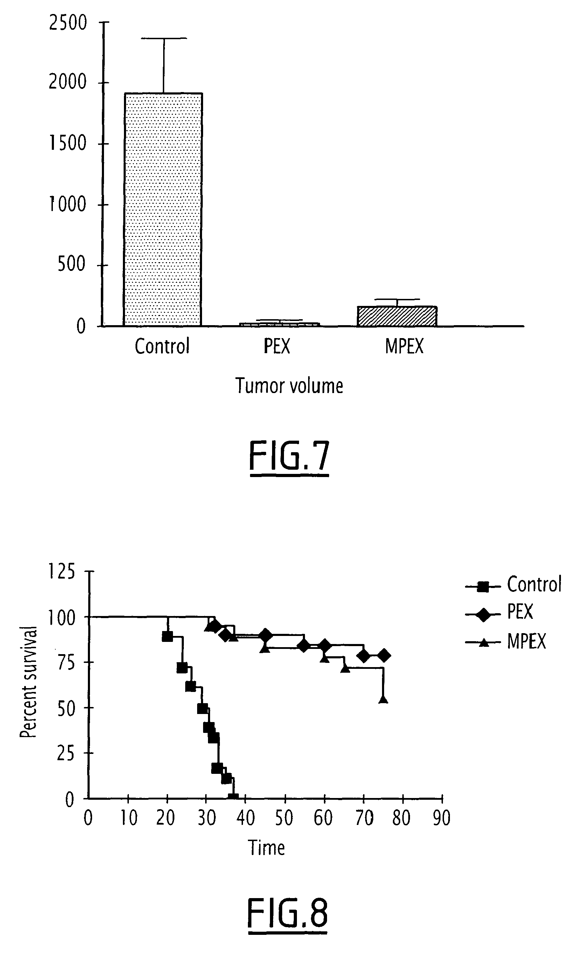

The ability of MPEX or human PEX to inhibit tumor growth in vivo was also investigated in the Tyrp1-TagSV40T mice model. In this model a tumor develops at birth from the retinal epithelium and progressively grows into the brain by invading the optic nerve, chiasm, and basal ganglia. This model allows studying the inhibitory effect of the molecules in a spontaneous tumor growth setting. The second advantage is that this model allows studying tumor cell invasion into the brain. When delivered systemically to these mice by subcutaneous minipumps starting 4 weeks after birth, both MPEX and human PEX were able to potently inhibit tumor growth. Treatment with MPEX was associated with a 97% tumor volume reduction. That with PEX induced a 99% decrease in tumor volume (Figure 7).

The ability of MPEX to sustain a long term inhibition of glioma growth in vivo was investigated by performing long term experiments in the U87 nude mice glioma model. In these experiments the effect exerted by MPEX was compared with that induced by human PEX. Both molecules were administered systemically and continuously by osmotic minipumps filled with 0.5mg/Kg of inhibitor. To afford a prolonged period of treatment the pumps were replaced three times. Treatment with both MPEX and human PEX significantly prolonged survival of treated animals. Fifty per cent survival of animals treated with MPEX and PEX was longer than 90 days, and at this time point 80 and 75% of the treated animals were still alive. Both treatments were well tolerated without the occurrence of any side effects (Figure 8).

### Histological analysis

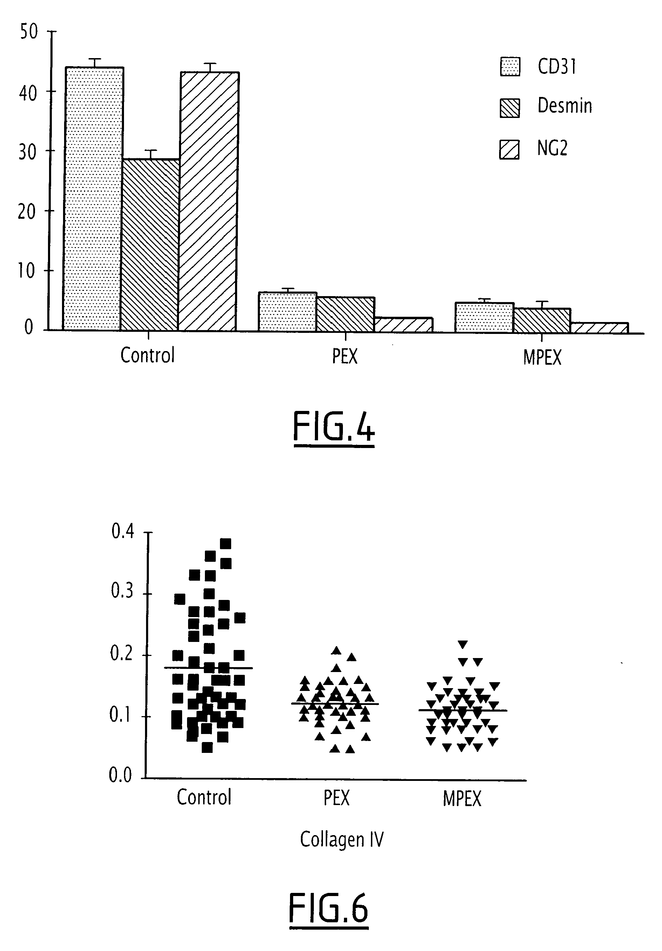

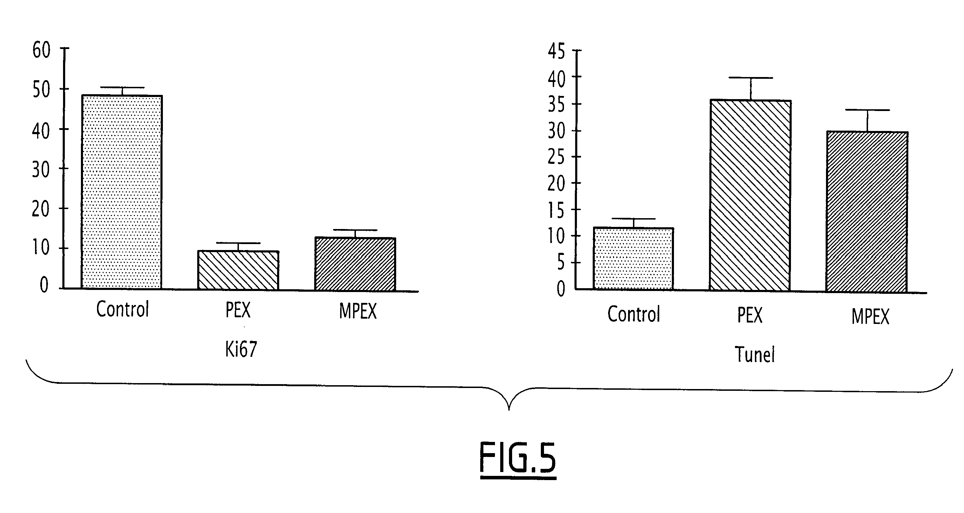

Histological analysis of glioma xenografts treated with MPEX and human PEX showed that the inhibition of tumor growth was associated with a marked decrease in vessel count, decrease in vessel diameters as well as increase in the intercapillary distance. The thickness of the basal membrane was reduced as well. Apoptotic rate was increased and proliferation rate was decreased (Figure 5). Analysis of pericyte coverage showed that treatment with both molecules was associated with a remodelling of tumor vasculature. While in tumors from control animals, vessels were composed by endothelial cells irregularly disposed in layers and inhomogeneously covered by pericytes, in tumors from animals treated with MPEX or PEX, the vasculature was formed of small elongated vessels composed of a unilayer of endothelial cells, homogeneously covered by pericytes, forming a continuous coverage. While in control tumors, endothelial cells were covered by PDGF Beta receptor, desmin, and NG2 positive pericytes, in those treated with MPEX or PEX, there was a significant decrease in the NG2 pericyte coverage (Figure 3 and 4). Collagen IV associated with vascular basal membranes is also decreased by both molecules (Figure 6). Similar findings were observed in the Tyrp1-TagSV40T mice model.

### REFERENCES

Abuchowski A, van Es T, Palczuk NC, Davis FF. Alteration of immunological properties of bovine serum albumin by covalent attachment of polyethylene glycol. J Biol Chem. 1977 Jun 10;252(11):3578-81
Ausprunk DH, Knighton DR, Folkman J. Vascularization of normal and neoplastic tissues grafted to the chick chorioallantois. Role of host and preexisting graft blood vessels. Am J Pathol. 1975 Jun;79(3):597-628
Bello L, Lucini V, Carrabba G, Giussani C, Machluf M, Pluderi M, Nikas D, Zhang J, Tomei G, Villani RM, Carroll RS, Bikfalvi A, Black PM. Simultaneous inhibition of glioma angiogenesis, cell proliferation, and invasion by a naturally occurring fragment of human metalloproteinase-2. Cancer Res. 2001 Dec 15;61 (24):8730-6
Brekken RA, Li C, Kumar S. Strategies for vascular targeting in tumors. Int. J. Cancer. 2002 May 23;100 (2): 123-130
Brooks PC, Clark RA, Cheresh DA. Requirement of vascular integrin alpha v beta 3 for angiogenesis. Science. 1994 Apr 22;264(5158):569-71
Brooks PC, Montgomery AM, Rosenfeld M, Reisfeld RA, Hu T, Klier G, Cheresh DA. Integrin alpha v beta 3 antagonists promote tumor regression by inducing apoptosis of angiogenic blood vessels. Cell. 1994 Dec 30;79(7):1157-64
Brooks PC, Silletti S, von Schalscha TL, Friedlander M, Cheresh DA. Disruption of angiogenesis by PEX, a noncatalytic metalloproteinase fragment with integrin binding activity. Cell. 1998 Feb 6;92(3):391-400
Chapman AP. PEGylated antibodies and antibody fragments for improved therapy: a review. Adv Drug Deliv Rev. 2002 Jun 17;54(4):531-45
Cunningham-Rundles C, Zhuo Z, Griffith B, Keenan J. Biological activities of polyethylene-glycol immunoglobulin conjugates. Resistance to enzymatic degradation. J Immunol Methods. 1992 Aug 10;152(2):177-90
D'Amato RJ, Loughnan MS, Flynn E, Folkman J. Thalidomide is an inhibitor of angiogenesis. Proc Natl Acad Sci U S A. 1994 Apr 26;91 (9):4082-5
Folkman J. How is blood vessel growth regulated in normal and neoplastic tissue? G.H.A. Clowes memorial Award lecture. Cancer Res. 1986 Feb;46(2):467-73
Friedlander M, Brooks PC, Shaffer RW, Kincaid CM, Varner JA, Cheresh DA. Definition of two angiogenic pathways by distinct alpha v integrins. Science. 1995 Dec 1;270(5241):1500-2
Kaspar M, Zardi L, Neri D. Fibronectin as target for tumor therapy. Int J Cancer. 2006 Mar 15;118(6):1331-9
Monfardini C, Schiavon O, Caliceti P, Morpurgo M, Harris JM, Veronese FM. A branched monomethoxypoly(ethylene glycol) for protein modification. Bioconjug Chem. 1995 Jan-Feb;6(1):62-9
Ossowski L, Reich E. Experimental model for quantitative study of metastasis. Cancer Res. 1980 Jul;40(7):2300-9
Pfeifer A, Kessler T, Silletti S, Cheresh DA, Verma IM. Suppression of angiogenesis by lentiviral delivery of PEX, a noncatalytic fragment of matrix metalloproteinase 2. Proc Natl Acad Sci U S A. 2000 Oct 24;97(22):12227-32
Pluderi M, Lucini V, Caronzolo D, Pannacci M, Costa F, Carrabba G, Giussani C, Grosso S, Colleoni F, Scaglione F, Villani R, Bikfalvi A, Bello L. Long-term inhibition of glioma growth by systemic administration of human PEX. J Neurosurg Sci. 2003 Jun;47(2):69-78
Varner JA, Brooks PC, Cheresh DA. REVIEW: the integrin alpha V beta 3: angiogenesis and apoptosis. Cell Adhes Commun. 1995 Nov;3(4):367-74
Weidner N, Semple JP, Welch WR, Folkman J. Tumor angiogenesis and metastasis--correlation in invasive breast carcinoma. N Engl J Med. 1991 Jan 3;324(1):1-8
Yan HC, Juhasz I, Pilewski J, Murphy GF, Herlyn M, Albelda SM. Human/severe combined immunodeficient mouse chimeras. An experimental in vivo model system to study the regulation of human endothelial cell-leukocyte adhesion molecules. J Clin Invest. 1993 Mar;91 (3):986-96

## Claims

1. An isolated polypeptide comprising the amino acid sequence ranging from positions 19 to 210 of SEQ ID NO:1, wherein at least one cysteine residue at position 19 or 210 of SEQ ID NO:1 has been substituted or deleted, or a function-conservative variant thereof.

2. A polypeptide according to claim 1, which comprises the amino acid sequence ranging from positions 13 to 210 of SEQ ID NO: 1, wherein at least one cysteine residue at position 19 or 210 of SEQ ID NO:1 has been substituted or deleted, or a function-conservative variant thereof.

3. A polypeptide according to claim 1 or 2, which comprises the amino acid sequence SEQ ID NO:1, wherein at least one cysteine residue at position 19 or 210 of SEQ ID NO:1 has been substituted or deleted, or a function-conservative variant thereof.

4. A polypeptide according to claim 1, which comprises the amino acid sequence ranging from positions 35 to 237 of SEQ ID NO:2, wherein at least one cysteine residue at position 46 or 237 of SEQ ID NO:2 has been substituted or deleted, or a function-conservative variant thereof.

5. A polypeptide according to claim 1 or 4, which comprises the amino acid sequence SEQ ID NO:2, wherein at least one cysteine residue at position 46 or 237 of SEQ ID NO:2 has been substituted or deleted, or a function-conservative variant thereof.

6. A polypeptide according to any of claims 1 to 5, wherein said at least one cysteine residue is substituted by an amino acid selected from the group consisting of asparagine, glutamine, serine, threonine, tyrosine, glycine, alanine, valine, leucine, isoleucine, proline, phenylalanine, methionine and tryptophane.

7. A polypeptide according to any of claims 1 to 6, wherein both cysteine residues are substituted or deleted.

8. A polypeptide according to claim 7, wherein both cysteine residues are substituted by a serine residue.

9. A polypeptide according to any of claims 1 to 8, which is covalently conjugated with at least one polyethylene glycol group.

10. A polypeptide according to any of claims 1 to 8, which is covalently coupled to a vascular or tumor targeting agent.

11. A nucleic acid comprising a sequence encoding a polypeptide according to any of claims 1 to 8.

12. A vector comprising a nucleic acid according to claim 11.

13. A host cell, which has been transformed by a nucleic acid according to claim 11 and/or a vector according to claim 12.

14. A method of producing a polypeptide according to any of claims 1 to 8, which method comprises the steps consisting of: (i) culturing a transformed host cell according to claim 13 under conditions suitable to allow expression of said polypeptide; and (ii) recovering the expressed polypeptide.

15. A pharmaceutical composition comprising a polypeptide according to any of claims 1 to 8, together with a pharmaceutically acceptable carrier.

16. A pharmaceutical composition comprising a nucleic acid according to claim 11 or a vector according to claim 12, together with a pharmaceutically acceptable carrier.

17. A pharmaceutical composition comprising a host cell according to claim 13, together with a pharmaceutically acceptable carrier.

18. Use of a polypeptide according to any of claims 1 to 8 for the manufacture of a medicament intended for treating an angiogenic disease.

19. Use of a nucleic acid according to claim 11 for the manufacture of a medicament intended for treating an angiogenic disease.

20. The use according to claim 18 or 19, wherein said medicament is intended for treating a condition selected from the group consisting of arthritis, rheumatoid arthritis, solid tumor metastasis, solid tumor, retinopathy, diabetic retinopathy or macular degeneration, psoriasis and chronic inflammatory skin diseases.
